# FASCICULE DE BREVET EUROPEEN

(11) **EP 3 370 663 B1**
(45) Date de publication et mention de la délivrance du brevet: **27.11.2019**
(21) Numéro de dépôt: 16794701.9
(22) Date de dépôt: 12.10.2016
(51) Int. Cl.: A61F 13/06, A61F 5/01

(54) **BANDE DE CONTENTION OU DE MAINTIEN D'UNE ARTICULATION**
BANDAGE ZUM RUHIGSTELLEN ODER HALTEN EINES GELENKS
BANDAGE FOR IMMOBILISING OR HOLDING A JOINT

(30) Priorité: 06.11.2015 FR 1560643
(43) Date de publication de la demande: 12.09.2018
(73) Titulaire: Millet Innovation, 26270 Loriol sur Drome (FR)
(72) Inventeur: MILLET, Damien, 26000 Valence (FR)
(74) Mandataire: de Roquemaurel, Bruno
(86) Numéro de dépôt international: PCT/FR2016/052630
(87) Numéro de publication internationale: WO 2017/077206

(56) Documents cités:
- WO-A1-2015/028734
- WO-A2-2007/113782
- DE-U1- 9 115 983

## Description

La présente invention concerne un dispositif de contention ou de maintien articulaire destiné à comprimer certaines zones limitées du corps, notamment en cas de faiblesse ligamentaire ou musculaire, ou d'une pathologie circulatoire, ou encore dans une situation post-opératoire. La présente invention s'applique notamment aux membres et aux articulations du genou, de la cheville, de l'épaule, du coude, du poignet, et des hanches.

Il existe des bandes de contention destinées à être utilisées dans des situations post-opératoires des membres inférieurs notamment pour limiter la formation d'hématomes, et/ou pour des pathologies circulatoires, ou encore pour prévenir des risques de lésions des tissus musculaires ou articulaires.

Il existe également des bandes pour le maintien du dos, conçues pour s'enrouler autour de la taille ou du buste en formant plusieurs tours qui se chevauchent. Ces bandes sont généralement réalisées en tissu élastique de type tricoté le plus souvent, avec un dispositif de serrage ajustable et un marquage permettant d'ajuster le serrage en fonction de recommandations du praticien. Ces bandes exercent une pression identique sur toute la zone couverte. Il s'avère également que ces bandes ont tendance à glisser le long du membre.

Cependant, dans plusieurs régions du corps il peut s'avérer nécessaire de pouvoir exercer une certaine pression de manière localisée en évitant des zones sensibles. Ainsi, dans l'exemple du pied, trois zones sont particulièrement sensibles à la pression. Il s'agit de la voussure dorsale du pied, de la zone péri-malléolaire tibiale et de la zone du tendon d'Achille. En effet, un serrage trop important de ces zones peut provoquer l'apparition de douleurs, voire de traumatismes. Il convient également de remarquer que la flexion dorsale de la cheville (pivotement du pied vers le haut) nécessaire à l'attaque d'un pas, est générée par la contraction des muscles releveurs du pied (tibial antérieur, court et long extenseurs des orteils). La contraction de ces muscles induit une saillance des tendons, en particulier celui du muscle tibial antérieur. Une pression trop importante exercée dans une telle zone de saillance du tendon de ce muscle peut donc provoquer une douleur insupportable et potentiellement une tendinopathie. Par ailleurs, une compression trop importante des artères et des nerfs (en particulier de l'artère pédieuse et des nerfs situés sur la voussure dorsale du pied) peut également provoquer un déficit d'oxygénation des tissus et des paresthésies. Dans la région péri-malléolaire tibiale, un serrage trop important peut également avoir des conséquences sur la circulation sanguine. Les bandes de contention ne permettent pas d'exercer un serrage suffisant des zones à maintenir tout en évitant que la pression exercée agisse également sur les zones sensibles.

Il existe des dispositifs se présentant sous la forme d'un bandage dont l'ajustement et le maintien serré est assuré par des éléments à boucles et à crochets (velcro®). Un tel bandage ne permet pas non plus d'exercer des pressions variables et ajustables en fonction de la zone couverte. Par ailleurs, outre leur aspect peu esthétique, les éléments à boucles et à crochets présentent une épaisseur et une rigidité relativement élevées, qui peuvent s'avérer gênantes au point d'entraver les mouvements, notamment si le bandage est porté sur une articulation et de surcroit dans une chaussure. L'épaisseur des éléments à boucles et à crochets peut également gêner ou empêcher le port d'un vêtement ajusté. Par ailleurs, les vêtements portés par-dessus le bandage peuvent être endommagés par l'élément à boucles dont les boucles peuvent s'accrocher aux mailles du tissu ou du tricot formant le vêtement.

L'ajustement de la pression exercée par un tel bandage s'avère également malaisé avec des éléments à boucles et crochets, car il ne peut pas être progressif. En effet, pour modifier cette pression, il est nécessaire de séparer entièrement les deux éléments à boucles et crochets pour les accrocher ensemble en un autre emplacement. Généralement, l'utilisateur doit ainsi réajuster la pression à plusieurs reprises avant d'atteindre la pression désirée.

Il existe également des dispositifs se présentant sous la forme de chaussettes ou de manchons tricotés, dont le tricotage est réalisé de manière sélective par anneau, afin d'exercer localement des pressions différentes selon la zone annulaire couverte. Ces dispositifs peuvent donc présenter localement une épaisseur relativement importante susceptible d'empêcher le port d'un vêtement ajusté. Ces dispositifs présentent également l'inconvénient d'imposer la fabrication d'un grand nombre de tailles différentes pour s'adapter aux différentes morphologies des utilisateurs.

Il existe également des bandes adhésives non élastiques ou de faible élasticité, destinées à être enroulées sur plusieurs tours autour d'un membre pour immobiliser partiellement une articulation ou comprimer un muscle, notamment dans un contexte sportif ou post-opératoire. Ces bandes adhésives présentent également l'inconvénient d'exercer une pression uniforme sur toutes les zones couvertes. En outre, elles sont nécessairement à usage unique.

Il est donc souhaitable de réaliser un dispositif de maintien d'une articulation ou de contention d'une partie d'un membre, qui puisse exercer sélectivement des pressions ajustables localement en fonction de la zone couverte. Il peut être également souhaitable que ce dispositif puisse être utilisé plusieurs fois et puisse s'adapter à diverses morphologies sans nécessiter la prévision d'un grand nombre de tailles différentes.

Des modes de réalisation concernent un dispositif de maintien ou de contention conformé pour entourer un membre ou une articulation d'un utilisateur, comprenant une bande élastique comportant deux tronçons de bande présentant des raideurs différentes, la bande élastique comportant une face interne en contact avec une zone couverte par la bande et une face externe opposée à la face interne, la face interne de la bande présentant, en présence d'une pression de contention exercée par la bande lorsque celle-ci est disposée sous tension autour d'un membre ou d'une articulation, une adhérence avec la zone couverte, telle que la bande puisse conserver localement des étirements résultant de forces d'étirement différentes, après disparition de ces forces d'étirement.

Selon un mode de réalisation, un premier des deux tronçons de bande comprend une plaquette en un matériau élastique, fixée par deux extrémités opposées à la bande,

Selon un mode de réalisation, la plaquette est fixée sur la face interne du dispositif de maintien.

Selon un mode de réalisation, la plaquette est fixée sur la face externe du dispositif de maintien.

Selon un mode de réalisation, la plaquette comprend une couche en matériau viscoélastique et une couche de tissu élastique fixée sur la couche en matériau viscoélastique.

Selon un mode de réalisation, la couche de tissu élastique présente une raideur plus faible dans le sens longitudinal de la bande que dans le sens transversal.

Selon un mode de réalisation, un premier des deux tronçons de bande présente une raideur comprise entre 2 et 4 fois celle d'un second des deux tronçons de bande.

Selon un mode de réalisation, la face interne est formée par une couche en un gel polymère tel que du PolyDiMethylSiloxane.

Selon un mode de réalisation, la bande élastique est conformée sous la forme d'un manchon.

Selon un mode de réalisation, la face interne de la bande présente, en présence d'une pression de contention exercée lorsque la bande est disposée sous tension autour d'un membre ou d'une articulation, une adhérence avec la zone couverte pouvant conserver des étirements résultant de forces d'étirement qui diffèrent de 20 N.

Selon un mode de réalisation, le dispositif comprend des première et seconde parties de bande conformées pour être enroulées autour de la cheville et du pied, la première partie de bande comprenant un premier tronçon de bande présentant une raideur plus élevée qu'un autre tronçon de bande de la première partie de bande, la seconde partie de bande comprenant une extrémité fixée sur la face externe de la première partie, un second tronçon de bande présentant une raideur plus élevée qu'un autre tronçon de bande de la seconde partie de bande, les premier et second tronçons de bande ayant une raideur plus élevée étant agencés sur les parties de bande pour pouvoir couvrir simultanément la malléole interne et des régions en dessous et au-dessus de la malléole externe de la cheville.

Des modes de réalisation peuvent également concerner un procédé de fabrication d'un dispositif de maintien ou de contention tel que précédemment défini, conformé pour entourer un membre ou une articulation d'un utilisateur.

Des modes de réalisation peuvent également concerner un procédé d'utilisation d'un dispositif de maintien tel que précédemment défini, le procédé d'utilisation comprenant des étapes consistant à : disposer le dispositif de maintien autour d'un membre ou une articulation, appliquer respectivement une force d'étirement plus élevée à un premier des tronçons de bande présentant une plus grande raideur qu'un second des tronçons de bande du dispositif de maintien, et une force d'étirement plus faible au second tronçon de bande du dispositif de maintien, pour étirer les premier et second tronçons, et supprimer les forces d'étirement.

Selon un mode de réalisation, le dispositif de maintien comprend des première et seconde parties de bande conformées pour être enroulées autour de la cheville et du pied, la première partie de bande comprenant un premier tronçon de bande présentant une raideur plus élevée qu'un autre tronçon de bande de la première partie de bande, la seconde partie de bande comprenant une extrémité fixée sur la face externe de la première partie, un second tronçon de bande présentant une raideur plus élevée qu'un autre tronçon de bande de la seconde partie de bande, le procédé comprenant des étapes consistant à : fixer une première extrémité de la première partie de bande à une orthèse de cheville comportant deux plaquettes rigides maintenues sur les malléoles de la cheville de l'utilisateur, enrouler, avec une première force de tension d'étirement, la partie de bande autour de la cheville, puis autour du pied en passant sur le dessus du pied, puis sous le pied où se trouve une extrémité du premier tronçon de bande, enrouler le premier tronçon de bande sur le pied, puis autour de la cheville en exerçant une seconde force de tension d'étirement supérieure à la première force de tension, pour étirer le premier tronçon de bande, de manière à serrer entre elles une partie inférieure d'une première des deux plaquettes, et une seconde des deux plaquettes, enrouler l'extrémité de la première partie de bande autour de la cheville avec une force de tension d'étirement inférieure à la seconde force de tension, et accrocher une seconde extrémité de la première partie de bande, enrouler avec une force de tension d'étirement inférieure à la première force de tension, la seconde partie de bande en passant sur le dessus du pied, puis autour de la cheville, le second tronçon de bande étant disposé sur une partie supérieure de la première plaquette, et accrocher une extrémité libre de la seconde partie de bande.

Des exemples de réalisation de l'invention seront décrits dans ce qui suit, à titre non limitatif en relation avec les figures jointes parmi lesquelles :
les figures 1 et 2 représentent schématiquement en coupe transversale et en vue de face, un dispositif de maintien, selon un mode de réalisation,
la figure 3 représente schématiquement en coupe transversale, un dispositif de maintien, selon un autre mode de réalisation,
les figures 4 et 5 représentent schématiquement en coupe transversale et en vue de face, un dispositif de maintien, selon un autre mode de réalisation,
la figure 6 représente schématiquement en coupe transversale, un dispositif de maintien, selon un autre mode de réalisation,
les figures 7 et 8 représentent des courbes de variation de la force d'étirement et de la raideur en fonction de l'allongement d'éléments constituant le dispositif de maintien,
les figures 9A, 9B sont des vues en coupe transversale schématiques du dispositif de maintien disposé autour d'un membre ou une articulation, dans deux configurations différentes,
la figure 10 représente schématiquement la face latérale externe d'un pied et d'une cheville, équipés d'une orthèse de cheville selon un mode de réalisation,
la figure 10A représente une pièce thermoformable de l'orthèse de cheville de la figure 10, selon un mode de réalisation,
la figure 11 représente schématiquement la face latérale interne du pied et de la cheville, équipés de l'orthèse montrée sur la figure 10,
la figure 11A représente une autre pièce thermoformable de l'orthèse de cheville de la figure 10, selon un mode de réalisation,
la figure 12 représente schématiquement en coupe transversale une bande de maintien, selon un mode de réalisation,
la figure 13 représente des courbes de variation de la force d'étirement appliquée à la bande de maintien de la figure 12, lors de son enroulement autour du pied et de l'orthèse de cheville.

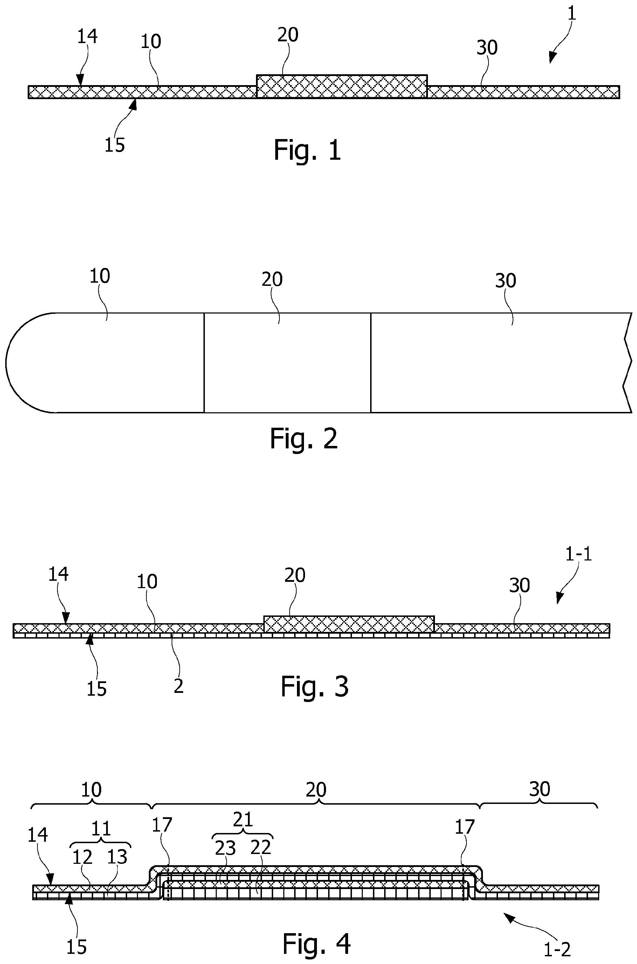

Les figures 1 et 2 représentent un dispositif de maintien 1 selon un mode de réalisation. Le dispositif de maintien 1 présente la forme d'une bande comprenant plusieurs tronçons de bande 10, 20, 30, élastiques présentant des raideurs différentes. A cet effet, les tronçons de bande 10, 20, 30 peuvent présenter des épaisseurs différentes, et/ou être formés dans des matériaux différents. Les tronçons de bande 10, 20, 30 peuvent être assemblés entre eux par tout moyen, tel que par des lignes de soudure ou de couture.

Le dispositif de maintien 1 présente une face externe 14 et face interne 15 opposée à la face 14 et destinée à venir en contact avec la zone couverte par le dispositif de maintien. Selon un mode de réalisation, la face interne 15 présente une certaine adhérence à la zone couverte sous l'effet d'une pression de contention exercée lorsque le dispositif de maintien 1 est disposé sous tension autour d'un membre ou d'une articulation. Cette adhérence à la zone couverte est telle que les tronçons de bande 10, 20, 30 puissent exercer des pressions différentes. Ces différentes pressions sont obtenues en soumettant individuellement à des forces d'étirement différentes les tronçons de bande 10, 20, 30 qui conservent au moins en partie leur étirement après disparition de ces forces grâce à l'adhérence de la face interne 15. Cette adhérence dépend de la surface de contact, de la courbure de celle-ci (lorsqu'elle est enroulée autour d'un membre) et de la nature du matériau sur la face interne 15 de chacun des tronçons de bande 10, 20, 30, ainsi que du matériau dans lequel est formé la zone couverte par le dispositif. Grâce à cette adhérence, il est possible d'obtenir des zones d'ancrage entre lesquelles une pression de contention plus élevée peut être exercée.

Ainsi le dispositif de maintien 1, lorsqu'il est disposé autour d'un membre ou d'une articulation, permet d'exercer une pression plus élevée sur les zones du membre couvertes par le tronçon de bande 20, et à l'inverse des pressions moins élevées sur les régions du membre couvertes par les tronçons 10, 30.

Dans l'exemple de la figure 1, le tronçon de bande 20 présente une raideur plus élevée que les tronçons de bande 10, 30. Il peut donc être étiré sous l'effet d'une relativement grande force d'étirement en s'allongeant moins que les tronçons 10, 30 si ces derniers étaient soumis à la même force d'étirement. Selon un exemple de réalisation, le tronçon de bande 20 présente une raideur comprise entre 2 et 4 fois la raideur des tronçons de bande 10, 30. Il en résulte que le tronçon de bande 20 s'étire entre 2 et 4 fois moins que les tronçons de bande 10, 30, sous l'effet d'une même force d'étirement.

Selon un mode de réalisation, l'adhérence de la face interne 15 du dispositif de maintien 1 est obtenue également par un choix approprié des matériaux dans lesquels sont formés les tronçons de bande 10, 20, 30. Selon un autre mode de réalisation, cette adhérence est obtenue en recouvrant la face interne 15 du dispositif 1 d'une couche élastique dans un matériau présentant l'adhérence souhaitée. Le choix des matériaux formant les différents tronçons de bande n'est donc pas limité par un critère d'adhérence. Ainsi, la figure 3 représente un dispositif de maintien 1-1 comprenant une couche élastique 2 recouvrant au moins en partie sa face interne 15 et présentant l'adhérence souhaitée. La couche 2 peut être réalisée par une enduction en un matériau viscoélastique répartie sur la face interne 15 en une couche par exemple d'épaisseur uniforme.

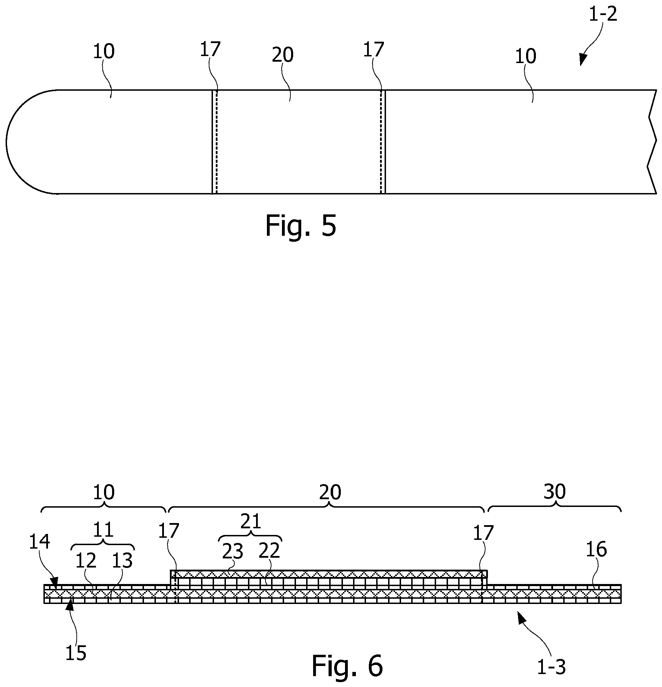

Les figures 4 et 5 représentent un dispositif de maintien 1-2 selon un mode de réalisation. Le dispositif de maintien 1-2 présente la forme d'une bande élastique comprenant les tronçons de bande 10, 20, 30 élastiques ayant des raideurs différentes. Les tronçons 10, 30 sont formés par une bande élastique 11 Le tronçon 20 est réalisé en fixant une plaquette 21 sur la couche 13 de la bande 11, par deux lignes de fixation 17 s'étendant le long de deux bords opposés de la plaquette 21. Ainsi, le tronçon de bande 20 présente une structure multicouches comprenant la bande 11 et la plaquette 21. Les lignes de fixation 17 sont formées sur la bande 11 suivant une direction transversale de la bande 11. Les lignes de fixation 17 sont formées de manière à ce que la plaquette 21 entre les deux lignes de fixation 17 présente une longueur correspondant sensiblement à celle de la partie de la bande 11 sous la plaquette entre les deux lignes de fixation 17, en l'absence d'étirement de la bande 11 et de la plaquette 21.

La bande 11 peut comprendre une couche 12 en un matériau élastique du côté de sa face externe 14 et une couche élastique 13 en un matériau présentant l'adhérence souhaitée du côté de sa face interne 15. La couche 12 peut être réalisée dans un tissu élastique au moins dans la direction longitudinale de la bande. La couche 13 peut être réalisée par une enduction en un matériau viscoélastique répartie sur la face interne 15 par exemple d'une manière uniforme. Les lignes de fixation 17 peuvent être formées par des lignes de couture. La plaquette 21 peut comprendre une couche 22 en un matériau viscoélastique formant la surface adhérente du tronçon 20, et une couche de tissu élastique 23 fixée sur la couche 22. La couche de tissu 23 peut être collée sur la couche 22. La couche 22 présente une certaine adhérence de manière à l'empêcher de glisser sur la surface couverte par le dispositif 1-2, en présence d'une certaine pression de contention. Cette adhérence est telle que le tronçon de bande 20 peut être étiré sous l'effet d'une force d'étirement différente de celles appliquées aux tronçons 10, 30, et conserver son étirement en l'absence de ces forces d'étirement, grâce à l'adhérence de la couche 22 et à la pression de contention exercée par le dispositif de maintien 1-2.

L'épaisseur de la bande 12 peut être choisie entre 0,3 et 1,5 mm, la couche 13 présentant une épaisseur comprise entre 0,1 et 0,5 mm. Les couches 2, 13 et 22 peuvent être réalisées dans des gels polymères viscoélastiques tels que des gels de silicone (PolyDiMethylSiloxane - PDMS) dont la dureté et l'adhérence sont choisis en fonction de leurs rôles respectifs dans le dispositif de maintien. L'épaisseur de de la couche 22 peut être choisie entre 0,3 et 1 mm.

Dans un mode de réalisation, la bande 11 présente une largeur comprise entre 5 et 6 cm, tandis que la plaquette 21 présente une largeur légèrement inférieure à celle de la bande 10, par exemple d'environ 2 à 7 mm.

Comme la couche adhérente 13 est répartie sur toute la surface de la bande 12, la plaquette 21 peut être fixée sur la face externe 14 de la bande 12, comme illustré sur la figure 6. Ainsi, la figure 6 représente un dispositif de maintien 1-3 qui diffère du dispositif de maintien 1-2 uniquement en ce que la plaquette 21 est fixée sur la face externe 14 de la bande 11. Il en résulte que la couche 13 formée sur la face interne de la bande 11 se trouve entièrement en contact avec la zone couverte par le dispositif de maintien 1-3.

Le dispositif de maintien 1, 1-1, 1-2, 1-3 peut être maintenu enroulé et étiré autour d'un membre ou d'une articulation, à l'aide d'un dispositif d'accrochage comprenant deux parties respectivement fixées aux extrémités de la bande 10 et coopérant entre elles pour se fixer l'une à l'autre. Ce dispositif d'accrochage peut par exemple comprendre une ou plusieurs boucles fixées à une extrémité du dispositif de maintien et des crochets fixés à une extrémité opposée du dispositif de maintien, ou bien des bandes à crochets et à boucles (velcro®) fixées aux extrémités opposées du dispositif de maintien.

Ainsi, le dispositif de maintien 1, 1-1, 1-2, 1-3 peut être enroulé autour d'un membre ou d'une articulation en formant plus d'un tour autour du membre, de sorte qu'une partie de la face interne 15 du dispositif de maintien se trouve en contact avec une partie de la face externe 14 du dispositif de maintien. Il peut alors être prévu de recouvrir au moins en partie la face externe 14 du dispositif de maintien d'une couche adhérente 16 (figure 6) qui peut être réalisée dans le même matériau que la couche adhérente 13. La partie de la face externe 14 recouverte de la couche 16 peut être limitée à la partie recouverte par la face externe 15 du dispositif, en raison de l'enroulement du dispositif sur plus d'un tour. Il va de soi que la couche 16 augmente la raideur des différents tronçons de bande où elle est présente.

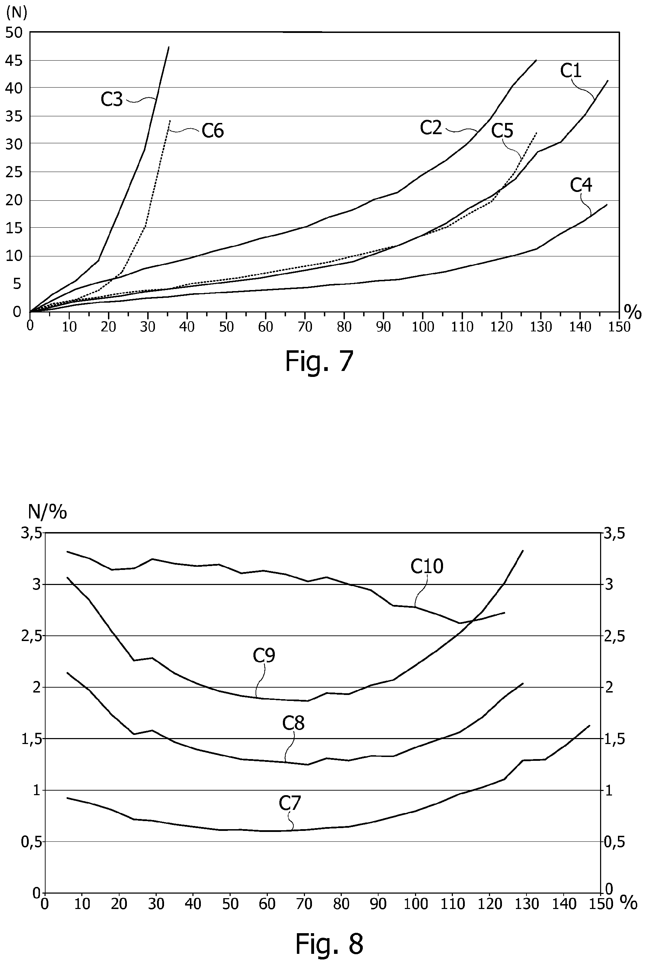

La figure 7 représente des courbes C1 à C6 représentatives de l'élasticité d'éléments constituant le dispositif de maintien 1-2 ou 1-3. Chacune des courbes C1 à C6 fournit les variations de la force d'étirement appliquée à l'élément en fonction d'un pourcentage d'allongement (en abscisse) de l'élément. La courbe C1 est représentative de l'élasticité longitudinale de la bande 11. La courbe C2 est représentative de l'élasticité longitudinale de la plaquette 21. La courbe C3 est représentative de l'élasticité transversale de la plaquette 21. La courbe C4 est représentative de l'élasticité longitudinale de la bande 12 seule. La courbe C5 est représentative de l'élasticité longitudinale de la couche de tissu 23 de la plaquette 21. La courbe C6 est représentative de l'élasticité transversale de la couche de tissu 23 de la plaquette 21. Les courbes C1 à C6 présentent sensiblement la forme de courbes hyperboliques passant par le point origine (de coordonnées 0%, 0 N). Les courbes C1 à C4 présentent en outre une région s'étendant entre 0 et 80% où l'allongement est sensiblement proportionnel à la force d'allongement exercée.

D'après les courbes C1 et C2, la bande 11 s'allonge d'environ de 147% et la plaquette 21 s'allonge d'environ 129%, sous l'effet de forces d'étirement respectivement de 41 N et 45 N. Les courbes C1 et C2 montrent notamment qu'entre des valeurs de force d'étirement de 5 et 40 N, la bande 11 s'allonge de 23 à 43% de plus que la plaquette 21. D'après la courbe C3, la plaquette 21 s'étire dans le sens transversal de 35% sous l'effet d'une force d'étirement de 47,5 N. La plaquette 21 présente donc dans la direction transversale une élasticité moindre que dans la direction longitudinale (courbes C2, C3). Cette disposition permet d'éviter que les régions de bord et de centre de la plaquette 21 ne s'étirent avec des allongements différents. Cette différence de raideur est conférée par la couche de tissu 23 composant la plaquette 21. En effet, d'après les courbes C5 et C6, la couche de tissu 23 s'étire dans la direction longitudinale de 124% et dans la direction transversale de 34%, sous l'effet d'une force d'étirement de 32 N. D'après la courbe C4, la bande 11 seule s'étire de 147% sous l'effet d'une force d'étirement de 19,5 N.

Selon un mode de réalisation, la couche de tissu 23 présente dans le sens où elle est fixée à la bande 11, une raideur de 2 à 4 fois plus élevée dans le sens transversal que dans le sens longitudinal de la bande 11.

La figure 8 représente des courbes de raideur C7, C8, C9, respectivement des tronçons de bande 10, 30 (bande 11) du dispositif de maintien 1-2 ou 1-3, de la plaquette 21 seule, et du tronçon 20 combinant la bande 11, et la plaquette 21. D'après la courbe C7, la raideur des tronçons 10, 30 varie entre 0,6 et 1,25 N/% lorsque l'allongement de ces tronçons est compris entre 5 et 130%. D'après la courbe C8, la raideur longitudinale de la plaquette 21 varie entre 1,25 et 2,1 N/% lorsque l'allongement longitudinal de la plaquette 21 est compris entre 5 et 130%. D'après la courbe C9, la raideur du tronçon de bande 20 (plaquette 21 + bande 11) varie entre 1,8 et 3,3 N/% lorsque l'allongement du tronçon de bande est compris entre 5 et 130%.

La figure 8 représente également une courbe C10 de variation du rapport des raideurs du tronçon de bande 20 et du tronçon 10 ou 30 du dispositif de maintien 1-2 ou 1-3. Ce rapport varie entre 2,6 et 3,3 pour des allongements variant entre 5 et 130%. La raideur du tronçon de bande 20 est donc en moyenne trois fois plus grande que la raideur des tronçons de bande 10 et 20 dans l'exemple du dispositif de maintien 1-2 ou 1-3. Grâce à la présence des tronçons 10, 30, le dispositif de maintien peut s'adapter à différentes tailles de membre ou d'articulation sans avoir à augmenter de manière sensible sa tension autour du membre ou de l'articulation, tout en ayant à ses extrémités des points d'accrochage fixes. A contrario, le tronçon de bande 20 peut recevoir une grande tension sans s'étirer d'une manière importante. Il peut ainsi restituer des pressions relativement élevées à la zone qu'il recouvre. Ainsi dans l'exemple des figures 7 et 8, le tronçon de bande 20 s'allonge environ trois fois moins que le tronçon de bande 10 ou 30 sous l'effet d'une même force de tension.

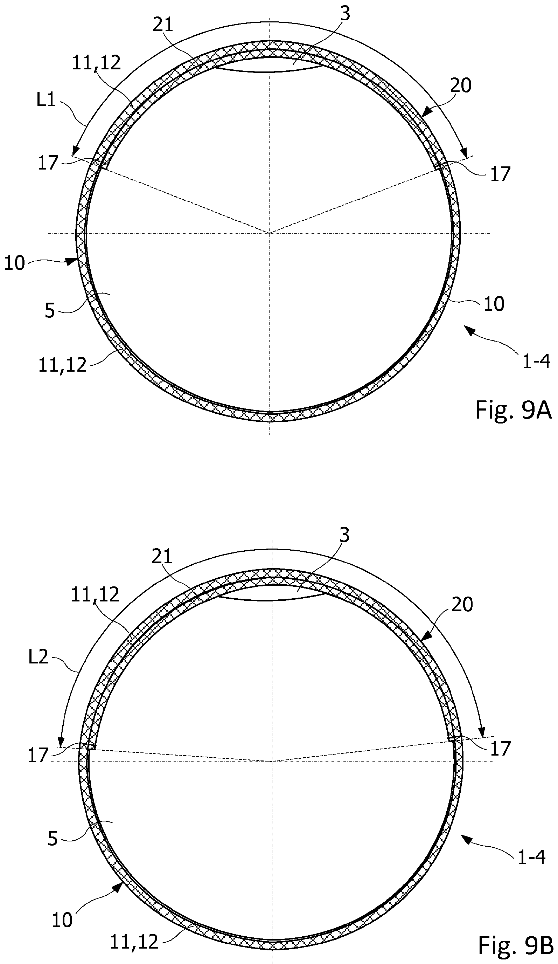

Selon un mode de réalisation, le dispositif de maintien est agencé sous la forme d'un manchon en fixant entre elles les deux extrémités opposées de la bande 11, comme illustré sur les figures 9A, 9B.

Les figure 9A, 9B représentent un dispositif de maintien 1-4, dans deux configurations différentes, pour illustrer un mode d'utilisation du dispositif de maintien. Il est à noter que ces deux configurations peuvent être obtenues de la même manière avec l'un ou l'autre des dispositifs de maintien 1, 1-1 à 1-3 en forme de bande, dès lors qu'ils sont fixés enroulés autour d'un membre ou d'une articulation. Dans l'exemple des figures 9A, 9B, le dispositif de maintien 1-4 comprend deux tronçons de bande 10, 20, le tronçon de bande 20 présentant une raideur plus grande que le tronçon de bande 10. Sur la figure 9A, le dispositif de maintien 1-4 a été disposé autour d'un membre ou d'une articulation 5 avec une tension uniforme sur tout le tour du membre. Dans la configuration de la figure 9A, le tronçon de bande 20 présente une longueur L1. Dans cette configuration, le dispositif de maintien 1-4 exerce sur la peau une certaine force de contention qui est sensiblement constante tout autour du membre ou de l'articulation 5, et qui est liée à l'allongement cumulé des tronçons de bande 10 et 20 compte tenu des raideurs différentes des deux tronçons de bande 10, 20.

Sur la figure 9B, le dispositif de maintien a été placé autour du membre ou de l'articulation en appliquant au tronçon de bande 20 une force de tension plus grande que la force de tension appliquée au tronçon de bande 10. Dans la configuration de la figure 9B, le tronçon de bande 20 atteint une longueur L2 supérieure à la longueur L1. Cette configuration est stable en l'absence de force de tension jusqu'à un certain point, grâce à l'adhérence de la face interne du tronçon de bande 20 avec la zone couverte par le dispositif de maintien 1-4, apparaissant en présence de la pression de contention exercée par le dispositif 1-4. La configuration du dispositif 1-4 sur la figure 9B peut être obtenue à partir de celle de la figure 9A, par exemple en pinçant avec une main chaque extrémité du tronçon de bande 20, et en étirant ce tronçon de bande autour du membre 5. Il en résulte que le tronçon de bande 10 présente un allongement dans la configuration de la figure 9B inférieur à celui de la configuration de la figure 9A, et donc qu'il exerce encore moins de pression.

Dans la configuration de la figure 9B, le tronçon de bande 20 exerce sur la zone du membre qu'il recouvre, une pression de contention plus élevée que celle exercée par le tronçon de bande 10 dans la configuration de la figure 9A ou 9B. Par ailleurs, le tronçon de bande 10 dans la configuration de la figure 9B exerce sur la zone du membre qu'il recouvre, une pression de contention moins élevée que celle exercée par le tronçon de bande 10 dans la configuration de la figure 9A. Le dispositif de maintien peut donc être disposé autour d'un membre ou d'une articulation de manière à y exercer des pressions de contention moins élevées dans une certaine zone couverte par le tronçon de bande 10, et plus élevées dans une autre zone couverte par le tronçon de bande 20.

Selon un mode de réalisation, le dispositif de maintien 1-4 comprend un coussinet 3 en un matériau viscoélastique fixé sur la face intérieure du dispositif, destiné à venir en contact avec la zone recouverte par le dispositif. Dans l'exemple des figures 9A, 9B, le coussinet 3 est fixé sur la face intérieure de la plaquette 21. Le coussinet 3 est prévu pour exercer une pression plus élevée en une zone localisée. Le coussinet 3 peut présenter une forme adaptée à la zone de la peau où une pression plus élevée doit être appliquée. La position du coussinet 3 peut être choisie par l'utilisateur. A cet effet, le coussinet 3 et la plaquette 21 peuvent être formés avec du gel de silicone (PDMS) présentant la propriété d'adhérer naturellement d'une manière détachable à une autre pièce réalisée dans ce matériau.

Le manchon 1-4 représenté sur les figures 9A, 9B peut être prévu pour contribuer à renforcer la liaison entre le périoste et l'os du tibia. A cet effet, le tronçon de bande 20 est placé sur le tibia, tandis que le tronçon de bande 10 couvre la partie postérieure de la jambe constituée de muscles et de tendons qu'il faut éviter de comprimer. En étirant le tronçon de bande 20 sous l'effet d'une force de tension plus grande que celle appliquée au tronçon de bande 10, le dispositif 1-4 exerce une pression plus élevée localisée sur la région antérieure de la jambe, tout en exerçant une pression faible sur la masse musculaire sur la région postérieure de la jambe. La pression exercée sur la région antérieure de la jambe favorise le maintien de la région tibia-périoste qui peut être fortement sollicitée notamment lors de la pratique de sports soumettant cette partie du corps à de fortes vibrations répétées et de fortes tractions appliquées en particulier sur les insertions musculaires présentes dans cette partie du corps. Les sports concernés sont notamment la course à pied, la danse, et les sports de raquette tels que le tennis et le squash. La présence du tronçon de bande 10 peu tendu est particulièrement importante car il faut éviter de produire un effet de garrot sur le mollet qui se gonfle fortement lorsqu'il est sollicité.

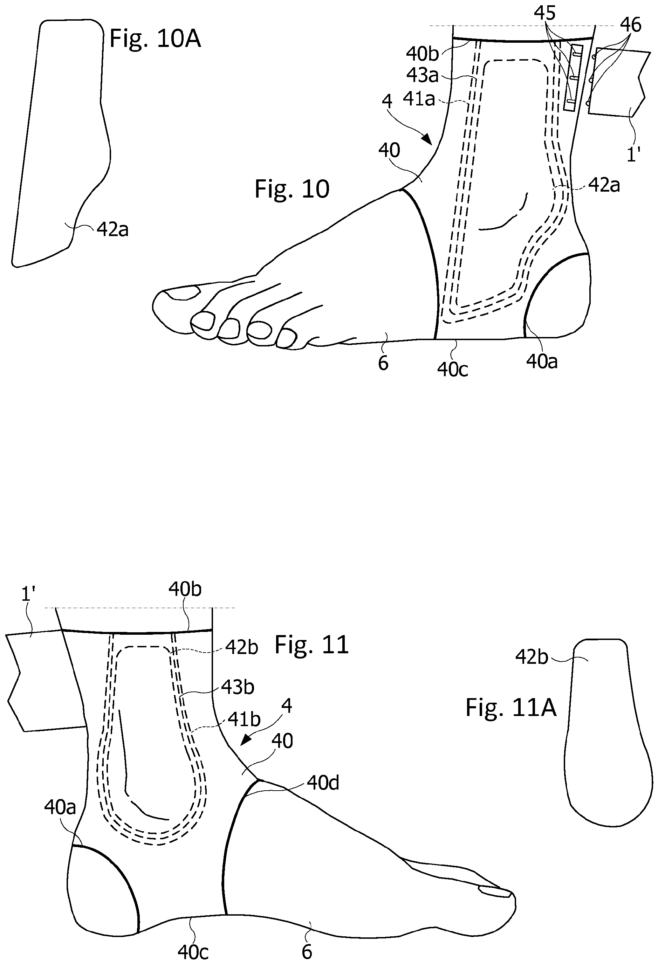

Les figures 10 et 11 représentent une orthèse 4 adaptée au maintien de la cheville, par exemple à la suite d'un traumatisme, ou présentant une laxité justifiant son maintien formel ou d'un point de vue proprioceptif. L'orthèse 4 comprend une couche 40 au moins en partie élastique, formant un manchon conformé pour serrer la cheville et le pied 6, avec une ouverture proximale 40b pour le passage du pied, puis de la jambe, une ouverture distale 40d pour le passage du pied 6 et une ouverture intermédiaire 40a pour le passage du talon. La couche 40 est au moins en partie élastique. Une couche 41a est fixée sur la couche 40 de manière à former une première poche dans laquelle est insérée une première plaquette 42a en un matériau thermoformable. La forme de la première poche est ajustée à celle de la plaquette 42a. Ainsi le taux de remplissage de la poche par la plaquette peut être compris entre 80 et 95%. La forme de la plaquette 42a peut être elle-même définie en fonction d'une zone du membre ou de l'articulation où le maintien est à assurer. La première poche est formée sur la couche 40 en un emplacement correspondant à la malléole externe et s'étendant latéralement entre les ouvertures 40a et 40d et entre l'ouverture proximale 40b de la couche 40 et une partie 40c de la couche 40 couvrant un bord latéral du pied 6 entre les zones plantaire et dorsale. La forme de la plaquette 42a est représentée sur la figure 10A. La première poche est formée en fixant la couche 41a sur la couche 40, par une ligne de fixation 43a. La première poche présente par exemple une ouverture le long de l'ouverture proximale 40b du manchon 40, pour insérer ou retirer la plaquette 42a de la poche. La couche 40 peut être mise en contact direct avec la peau 6 de l'utilisateur. La couche 41a peut être fixée sur la couche 40 par une ligne de soudure ou de couture 43a, sur l'une ou l'autre des faces de la couche 40.

Selon un mode de réalisation, l'orthèse 4 comprend une seconde plaquette 42b en un matériau thermoformable disposée dans une seconde poche formée sur la couche 40 en un emplacement destiné à couvrir la malléole interne et s'étendant latéralement sur la couche 40 autour de la malléole interne jusqu'à l'ouverture 40b du manchon. La forme de la plaquette 42b est représentée sur la figure 11A. La seconde poche 42b est formée à l'aide d'une couche de tissu 41b fixée sur le manchon 40, par une ligne de fixation 43b. La seconde poche peut également présenter une ouverture le long de l'ouverture proximale 40b du manchon 40 pour insérer ou retirer la plaquette 42b de la poche.

Il est à noter que les figures 10A et 11A montrent la forme des plaquettes 42a, 42b avant leur thermoformage à chaud pratiqué directement sur le pied. Après l'opération de thermoformage, les plaquettes 42a, 42b prennent la forme exacte des malléoles et de la zone autour de ces dernières.

Une bande 1' (dont une partie est représentée sur les figures 10, 11) prévue pour être enroulée et serrée autour du pied 6 et de la cheville, peut être utilisée pour serrer davantage les plaquettes 42a, 42b contre la cheville, et ainsi pour solidariser l'ensemble des plaquettes 42a, 42b et de la cheville. La bande 1' peut être cousue ou accrochée à l'orthèse 4 par un dispositif d'accrochage comprenant par exemple un ou plusieurs crochets 45 sur l'orthèse 4, coopérant chacun avec une boucle 46 dans une région d'extrémité de la bande 1'.

L'orthèse 4 peut être utilisée de la manière suivante. Avant une première utilisation, l'orthèse doit subir une opération de thermoformage. A cet effet, elle est chauffée, par exemple en l'immergeant avec les plaquettes 42a, 42b dans leurs poches respectives, dans de l'eau chaude à une température suffisante pour ramollir les plaquettes 42a, 42b. A partir d'un certain moment après son contact avec l'eau chaude, les plaquettes 42a, 42b deviennent molles. Avant que les plaquettes 42a, 42b retrouvent leur rigidité, l'utilisateur enfile l'orthèse 4 autour de la cheville à maintenir. La tension élastique de la couche 40 plaque les plaquettes 42a, 42b contre la peau de l'utilisateur. Les plaquettes 42a, 42b prennent alors la forme des zones où elles sont plaquées, puis durcissent au bout de quelques minutes. Pendant le durcissement des plaquettes 42a, 42b, la cheville à maintenir est tenue dans la position finale souhaitée. L'orthèse 4 est ainsi adaptée à la morphologie de la zone où elle est appliquée, par le thermoformage des plaquettes 42a, 42b, réalisé simplement en enfilant l'orthèse sur la cheville, les forces élastiques exercées par la couche 40 assurant le maintien et la déformation des plaquettes 42a, 42b préalablement ramollies. Cette opération ne nécessite donc pas l'intervention d'une autre personne et en particulier d'un professionnel.

Avant l'opération de thermoformage, la partie du membre ou de l'articulation à maintenir peut être recouverte d'un film tel qu'un film plastique pour faciliter le retrait de l'orthèse mouillée à l'issue de l'opération de thermoformage.

Les plaquettes 42a, 42b sont par exemple réalisées en un matériau tel que "Aquaplast" fabriqué par la société Patterson. Elles présentent une épaisseur comprise entre 1,5 et 5 mm, par exemple environ 2,4 mm. Ce matériau devient mou à une température comprise entre 65 et 75°C, et reste malléable pendant environ quatre minutes. Ainsi, l'opération de thermoformage des plaquettes 42a, 42b peut être répétée autant de fois que nécessaire.

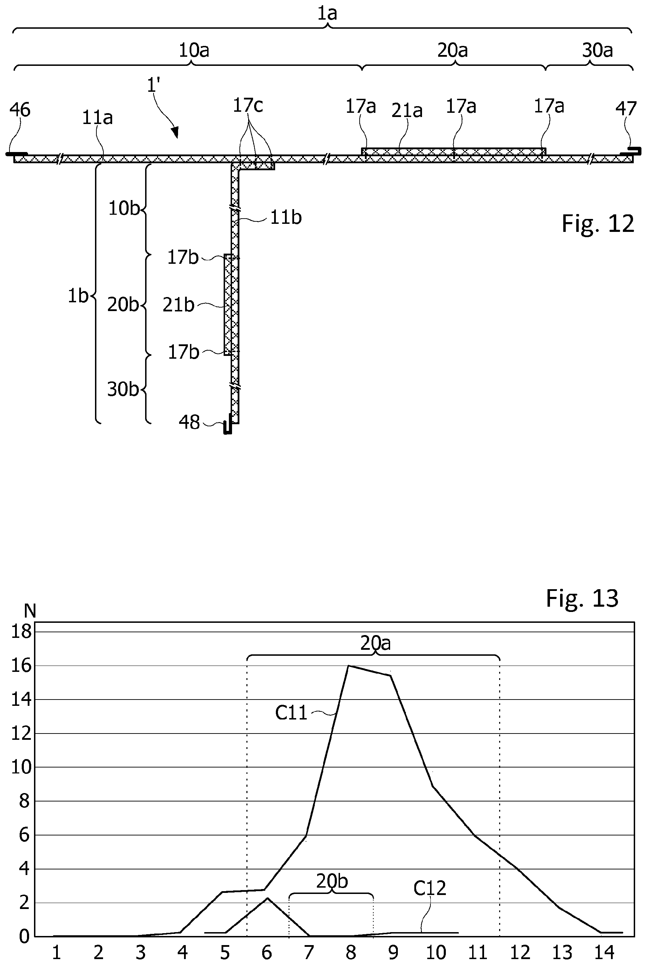

La figure 12 représente un exemple de réalisation de la bande 1' susceptible de s'accrocher à l'orthèse de cheville 4. La bande 1' est configurée pour s'enrouler autour de la cheville et du pied 6. A cet effet, la bande 1' comprend une partie principale 1a et une partie secondaire 1b fixée à la partie principale 1a, la bande 1' présentant ainsi une forme générale en T. La partie de bande 1b permet de recouvrir partiellement la partie 1a, et ainsi, renforcer le maintien assuré par cette dernière. Les parties de bande 1a, 1b présentent une structure telle que celle du dispositif 1. Dans l'exemple de la figure 12, les parties de bande 1a, 1b présentent la structure du dispositif 1-2. Ainsi, la partie principale 1a comprend des tronçons de bande 10a, 20a, 30a de raideurs différentes, tels que les tronçons de bande 10, 20, 30, une plaquette 21a telle que la plaquette 21, formant le tronçon de bande 20a avec la partie de bande 1a. La plaquette 21a est fixée sur la face interne de la partie de bande 1a par exemple par des lignes de couture 17a. La partie de bande secondaire 1b comprend également des tronçons de bande 10b, 20b, 30b de raideurs différentes, tels que les tronçons de bande 10, 20, 30, une plaquette 21b telle que la plaquette 21, formant le tronçon 20b avec la partie de bande 1b. La plaquette 21b est fixée sur la face interne de la partie de bande 1b par exemple par des lignes de couture 17b. Une extrémité de la partie de bande 1b est fixée par exemple par des lignes de couture 17c, sur la face externe de la partie de bande 1a opposée à la face interne où est fixée la plaquette 21a.

Une extrémité de la partie de bande 1a est tout d'abord accrochée à l'orthèse 4 par exemple au moyen de boucles 46 et de crochets 45 situés sur une partie supérieure de l'orthèse dans la région inférieure du mollet. La bande 1' peut également être cousue à l'orthèse 4. Dans un premier temps, la partie de bande 1a est enroulée sans tension autour de la cheville sur un peu plus d'un demi-tour en passant derrière celle-ci, puis autour du pied en passant sur le dessus du pied, et enfin sous le pied. Ensuite, l'utilisateur peut poser son pied au sol de manière à maintenir en place une extrémité du tronçon 20a de la partie de bande 1a, puis enroule le tronçon 20a en exerçant une plus grande tension sur l'extrémité libre de la partie de bande 1a. Le tronçon 20a est alors légèrement étiré en couvrant une partie inférieure de la plaquette 42a qu'il plaque sur le pied. Ensuite, en remontant, il vient couvrir la partie supérieure de la voussure du pied, peu sensible aux pressions, en évitant de passer sur la partie inférieure de la voussure très sensible où il pourrait entraver la flexion dorsale du pied. Enfin, le tronçon 20a couvre la majeure partie de la plaquette 42b (et en particulier la partie de la plaquette 42b couvrant la malléole interne). Une fois que le tronçon 20a est enroulé autour de la cheville, la tension sur l'extrémité libre de la partie de bande 1a est relâchée et son extrémité libre est accrochée à proximité des boucles 46 par un autre dispositif d'accrochage à crochet et à boucle 47 prévu à cet effet. En raison de l'adhérence de la face interne du tronçon 20a, celui-ci garde son allongement, notamment pendant que l'extrémité de la bande 1a est accrochée. Le tronçon de bande 20a crée ainsi une liaison forte entre les deux plaquettes 42a, 42b.

L'extrémité de la partie de bande 1b fixée à la partie de bande 1a se trouve alors parallèle à la face inférieure du pied sur la face latérale intérieure du pied. La partie de bande 1b est enroulée sans tension autour de la cheville sur un peu plus qu'un demi-tour en passant sur le pied, puis derrière la cheville, de manière à ce que la plaquette 21b se trouve en contact avec la partie de bande 1a. L'extrémité libre de la partie de bande 1b est ensuite accrochée sur la partie de bande 1a à proximité des boucles 46, au moyen d'un autre dispositif d'accrochage par exemple à crochet et à boucle 48. Dans cette configuration de la partie de bande 1b, le tronçon de bande 20b couvre la partie supérieure de la plaquette 42a, en la maintenant serrée contre la cheville.

La figure 13 représente des courbes C11, C12 de variation de la force de tension de la bande 1' après son enroulement autour du pied et de la chevillière, le long de la bande. Les tronçons de bande 1a, 1b ont été divisés en tronçons numérotés, de longueurs identiques avant étirement, les numéros de tronçons étant présentés en abscisse. Ainsi, sur la figure 13, la partie de bande 1a correspondant à la courbe C11, s'étend sur les tronçons numérotés de 1 à 14, le tronçon de bande 20a s'étend sur les tronçons numérotés de 6 à 11. La partie de bande 1b correspondant à la courbe C12, est fixée à la partie de bande 1a entre les tronçons portant les numéros 4 et 5, et s'étend sur les tronçons numérotés de 5 à 10, et le tronçon de bande 20b s'étend sur les tronçons numérotés 7 et 8.

D'après la courbe C11, la partie de bande 1a est enroulée sans tension jusqu'au tronçon numéro 3. Jusqu'au tronçon numéro 4, la force de tension exercée par la partie de bande 1a augmente légèrement de 0 à 0,2 N. Cette force de tension augmente encore pour atteindre 2,72 N au tronçon numéro 6 correspondant au début du tronçon 20a. Le tronçon 20a exerce une force de tension qui augmente progressivement pour atteinte une valeur voisine de 16 N au voisinage des tronçons numéro 8 et 9. La force de tension exercée par la partie de bande 1a diminue ensuite progressivement pour atteindre une valeur voisine de zéro à l'extrémité de la bande 1a au tronçon numéro 14. A la jonction avec le tronçon de bande 20a, le tronçon de bande 10a atteint un étirement d'environ 15%. L'étirement du tronçon de bande 20a augmente progressivement de 8 à 45% entre les tronçons numéro 6 et 8, puis diminue pour atteindre 15% au tronçon numéro 11. L'étirement du tronçon de bande 30a diminue progressivement de 25% à 5% entre les tronçons numéro 12 et 14.

D'après la courbe C12, la partie de bande 1b est enroulée pour atteindre une légère force de tension de 0,2 N qui augmente jusqu'à 2,25 N au tronçon numéro 6. Cette force de tension est nulle ou voisine de zéro le long du tronçon de bande 20b, et atteint 0,2 N à l'extrémité de la partie de bande 1b au tronçon numéro 10. L'allongement du tronçon de bande 10b passe de 5 à 13% entre les tronçons numéro 5 et 6. L'allongement de la partie de bande 20b est nul, et l'allongement de la partie 30b est de 5%.

Lorsque la bande est enroulée autour de l'orthèse et du pied, le tronçon numéro 9 (partie médiane de la plaquette 21a) recouvre les tronçons numéro 3 et 4 de la partie de bande 1a. Les tronçons numéro 7 et 8 (plaquette 21b) recouvrent les tronçons numéro 9 et 14 de la partie de bande 1a. Le tronçon numéro 9 de la partie de bande 1b recouvre le tronçon numéro 12 de la partie de bande 1a. Les forces de compression exercées par les différents tronçons superposés se cumulent.

Comme le tronçon de bande 20b n'est pas disposé avec un allongement, la plaquette 21b peut être réalisée dans un matériau non élastique. Le long de la bande 1', la conservation des différentes forces de tension exercées, qui peuvent différer de 20 N, est assurée par l'adhérence de la bande sur elle-même et sur la chevillière.

Les tronçons 20a, 20b de la bande 1', plus serrés et de raideur plus élevée, permettent ainsi de maintenir serrées contre la cheville les plaquettes 42a, 42b comme le ferait une attelle de cheville, le tronçon 20b complétant l'action du tronçon 20a pour assurer un maintien de la cheville en trois points, à savoir en bas et en haut de la plaquette 42a et sur la plaquette 42b. La bande 1' n'exerce pas de pressions trop élevées sur la voussure du pied et sur la zone tibiale inférieure juste au-dessus des malléoles qui sont couvertes par les tronçons 10a, 30a, 10b, 30b, moins serrés et de raideur moins élevée. En outre, les tronçons 10a, 30a, 10b, 30b peuvent être étirés plus ou moins pour placer correctement les tronçons 20a, 20b sur les plaquettes 42a, 42b en fonction de la morphologie de l'utilisateur, sans variation sensible de la pression qu'ils exercent sur les zones de le cheville qu'ils couvrent.

Les extrémités des parties de bande 1a, 1b supportant les boucles et les crochets 45 à 47 peuvent être renforcées par des pièces de tissu non élastique.

Il apparaîtra clairement à l'homme de l'art que la présente invention est susceptible de diverses variantes de réalisation et diverses applications. En particulier, l'invention n'est pas limitée aux modes de réalisation décrits, mais s'étend également à toutes les combinaisons possibles de ces modes de réalisation.

Par ailleurs, l'adhérence de la face interne 15 du dispositif 1, 1-1, 1-3, 1-4, de la bande 11 du dispositif 1-2, ou de la bande 1' peut être obtenue par des éléments viscoélastiques tels que des picots, répartis sur la face 15 par exemple d'une manière uniforme. La densité de ces éléments viscoélastiques sur la face interne 15 peut être choisie en fonction de l'adhérence à obtenir compte tenu de la pression de contention exercée par le dispositif et de l'adhérence de chaque élément viscoélastique.

Bien que les exemples décrits s'appliquent à la sécurisation du périoste et au maintien de la cheville, il va de soi que l'invention peut s'appliquer à toute partie du corps nécessitant l'application sélective d'une pression en une ou plusieurs zones distinctes.

## Revendications

1. Dispositif de maintien ou de contention conformé pour entourer un membre ou une articulation d'un utilisateur, comprenant une bande élastique (1) comportant deux tronçons de bande (10, 20) présentant des raideurs différentes, la bande élastique comportant une face interne (15) en contact avec une zone couverte par la bande et une face externe (14) opposée à la face interne, la face interne de la bande présentant, en présence d'une pression de contention exercée par la bande lorsque celle-ci est disposée sous tension autour d'un membre ou d'une articulation, une adhérence avec la zone couverte, telle que la bande puisse conserver localement des étirements résultant de forces d'étirement différentes, après disparition de ces forces d'étirement.

2. Dispositif selon la revendication 1, dans lequel un premier des deux tronçons de bande comprend une plaquette (21) en un matériau élastique, fixée par deux extrémités opposées à la bande (11),

3. Dispositif selon la revendication 2, dans lequel la plaquette (21) est fixée sur la face interne (15) du dispositif de maintien (1).

4. Dispositif selon la revendication 2, dans lequel la plaquette (21) est fixée sur la face externe (14) du dispositif de maintien (1).

5. Dispositif selon l'une des revendications 1 à 4, dans lequel la plaquette (21) comprend une couche en matériau viscoélastique (22) et une couche de tissu élastique (23) fixée sur la couche en matériau viscoélastique.

6. Dispositif selon la revendication 5, dans lequel la couche de tissu élastique (23) présente une raideur plus faible dans le sens longitudinal de la bande (1) que dans le sens transversal.

7. Dispositif selon l'une des revendications 1 à 6, dans lequel un premier des deux tronçons de bande (10, 20) présente une raideur comprise entre 2 et 4 fois celle d'un second des deux tronçons de bande.

8. Dispositif selon l'une des revendications 1 à 7, dans lequel la face interne (15) est formée par une couche en un gel polymère tel que du PolyDiMethylSiloxane.

9. Dispositif selon l'une des revendications 1 à 8, dans lequel la bande élastique est conformée sous la forme d'un manchon (1-4).

10. Dispositif selon l'une des revendications 1 à 9, dans lequel la face interne de la bande (1, 1') présente, en présence d'une pression de contention exercée lorsque la bande est disposée sous tension autour d'un membre ou d'une articulation, une adhérence avec la zone couverte pouvant conserver des étirements résultant de forces d'étirement qui diffèrent de 20 N.

11. Dispositif selon l'une des revendications 1 à 10, comprenant des première et seconde parties de bande (1a, 1b) conformées pour être enroulées autour de la cheville et du pied, la première partie de bande (1a) comprenant un premier tronçon de bande (20a) présentant une raideur plus élevée qu'un autre tronçon de bande (10a, 30a) de la première partie de bande, la seconde partie de bande (1b) comprenant une extrémité fixée sur la face externe de la première partie, un second tronçon de bande (20b) présentant une raideur plus élevée qu'un autre tronçon de bande (10b, 30b) de la seconde partie de bande, les premier et second tronçons de bande ayant une raideur plus élevée étant agencés sur les parties de bande pour pouvoir couvrir simultanément la malléole interne et des régions en dessous et au-dessus de la malléole externe de la cheville.

12. Procédé de fabrication d'un dispositif de maintien ou de contention conformé pour entourer un membre ou une articulation d'un utilisateur, selon l'une des revendications 1 à 11, le procédé comprenant des étapes d'assemblage de tronçons de bande (10, 20) élastique pour obtenir des tronçons de bande de raideurs différentes.

## Patentansprüche

1. Halte- oder Rückhaltevorrichtung, die zum Umgeben einer Gliedmaße oder eines Gelenks eines Benutzers angepasst ist, umfassend ein elastisches Band (1) mit zwei Bandabschnitten (10, 20) mit unterschiedlichen Steifigkeitsgraden, wobei das elastische Band eine Innenfläche (15), die mit einem von dem Band abgedeckten Bereich in Kontakt steht, sowie eine der Innenfläche gegenüberliegende Außenfläche (14) aufweist, wobei die Innenfläche des Bandes bei Vorliegen eines von dem Band ausgeübten Rückhaltedrucks, wenn dieses unter Spannung um eine Gliedmaße oder ein Gelenk gelegt wird, eine Haftung zu dem abgedeckten Bereich aufweist, sodass das Band die Dehnungen, die sich aus unterschiedlichen Dehnungskräften ergeben, lokal zurückhalten kann, nachdem diese Dehnungskräfte weggefallen sind.

2. Vorrichtung nach Anspruch 1, wobei ein erster der beiden Bandabschnitte eine Platte (21) aus elastischem Material umfasst, die an zwei dem Band (11) gegenüberliegenden Enden befestigt ist,

3. Vorrichtung nach Anspruch 2, bei der die Platte (21) an der Innenseite (15) der Haltevorrichtung (1) befestigt ist.

4. Vorrichtung nach Anspruch 2, bei der die Platte (21) an der Außenseite (14) der Haltevorrichtung (1) befestigt ist.

5. Vorrichtung nach einem der Ansprüche 1 bis 4, wobei die Platte (21) eine Schicht aus viskoelastischem Material (22) und eine Schicht aus elastischem Gewebe (23) umfasst, die an der Schicht aus viskoelastischem Material befestigt ist.

6. Vorrichtung nach Anspruch 5, wobei die Schicht aus elastischem Gewebe (23) in Längsrichtung des Bandes (1) eine geringere Steifigkeit als in Querrichtung aufweist.

7. Vorrichtung nach einem der Ansprüche 1 bis 6, wobei ein erster der beiden Bandabschnitte (10, 20) eine Steifigkeit zwischen dem 2- und 4-fachen eines zweiten der beiden Bandabschnitte aufweist.

8. Vorrichtung nach einem der Ansprüche 1 bis 7, wobei die Innenseite (15) aus einer Polymergel-Schicht, wie beispielsweise Polydimethylsiloxan, gebildet ist.

9. Vorrichtung nach einem der Ansprüche 1 bis 8, wobei das elastische Band in Form einer Hülse (1-4) geformt ist.

10. Vorrichtung nach einem der Ansprüche 1 bis 9, bei der die Innenseite des Bandes (1, 1') bei Vorliegen eines Rückhaltedrucks, der ausgeübt wird, wenn das Band unter Spannung um eine Gliedmaße oder ein Gelenk gelegt wird, eine Haftung zu der abgedeckten Fläche aufweist, welche Dehnungen aufgrund von Dehnungskräften zurückhalten kann, die sich um 20 N unterscheiden.

11. Vorrichtung nach einem der Ansprüche 1 bis 10, umfassend einen ersten und einen zweiten Bandteil (1a, 1b), die so angepasst sind, dass sie um Knöchel und Fuß gewickelt werden können, wobei der erste Bandteil (1a) einen ersten Bandabschnitt (20a) mit einer höheren Steifigkeit als ein anderer Bandabschnitt (10a, 30a) des ersten Bandteils umfasst, wobei der zweite Bandteil (1b) ein Ende umfasst, das an der Außenfläche des ersten Teils befestigt ist, wobei ein zweiter Bandabschnitt (20b) eine höhere Steifigkeit als ein anderer Bandabschnitt (10b, 30b) des zweiten Bandteils aufweist, und wobei der erste und zweite Bandabschnitt, die eine höhere Steifigkeit aufweisen, auf den Bandabschnitten angeordnet sind, um gleichzeitig den Innenknöchel sowie Bereiche unterhalb und oberhalb des Außenknöchels des Knöchels abzudecken.

12. Verfahren zur Herstellung einer Halte- oder Rückhaltevorrichtung, die zum Umgeben einer Gliedmaße oder eines Gelenks eines Benutzers angepasst ist, nach einem der Ansprüche 1 bis 11, wobei das Verfahren die Arbeitsschritte des Zusammensetzens von elastischen Bandabschnitten (10, 20) zum Erhalten von Bandabschnitten mit unterschiedlichen Steifigkeitsgraden umfasst.

## Claims

1. A supporting or compressing device configured to surround a limb or a joint of a user, comprising an elastic band (1) having two band segments (10, 20) having different stiffness values, the elastic band having an inner face (15) in contact with an area covered by the band and an outer face (14) opposite to the inner face, the inner face of the band providing, in the presence of a compression pressure exerted by the band when it is fitted under traction around a limb or joint, an adhesion with the covered area, such that the band conserves locally elongations resulting from different traction forces, after removal of the traction forces.

2. The device according to claim 1, wherein a first of the two band segments comprises a pad (21) made of an elastic material, attached by two opposite ends to the band (11),

3. The device according to claim 2, wherein the pad (21) is attached to the inner face (15) of the supporting device (1).

4. The device according to claim 2, wherein the pad (21) is attached to the outer face (14) of the supporting device (1).

5. The device according to one of claims 1 to 4, wherein the pad (21) comprises a layer of viscoelastic material (22) and a layer of elastic fabric (23) attached to the layer of viscoelastic material.

6. The device according to claim 5, wherein the elastic fabric layer (23) has a lower stiffness in the longitudinal direction of the band (1) than in the transverse direction.

7. The device according to one of claims 1 to 6, wherein a first of the two band segments (10, 20) has a stiffness between 2 and 4 times that of a second of the two band segments.

8. The device according to one of claims 1 to 7, wherein the inner face (15) is formed by a layer of a polymer gel such as Polydimethylsiloxane.

9. The device according to one of claims 1 to 8, wherein the elastic band is configured in the form of a sleeve (1-4).

10. The device according to one of claims 1 to 9, wherein the inner face of the band (1, 1') has, in the presence of a compression pressure exerted when the band is placed under traction around a member or a joint, an adhesion with the covered area which can retain elongations resulting from stretching forces that differ by 20 N.

11. The device according to one of claims 1 to 10, comprising first and second band portions (1a, 1b) configured to be wound around the ankle and foot, the first band portion (1a) including a first band segment (20a) having a higher stiffness than another band segment (10a, 30a) of the first band portion, the second band portion (1b) having an end fixed to the outer face of the first portion, a second band segment (20b) having a higher stiffness than another band segment (10b, 30b) of the second band portion, wherein the first and second band segments of higher stiffness are arranged on the band portions so that they can simultaneously cover the internal malleolus and regions below and above the external malleolus of the ankle.

12. A method of manufacturing a supporting or compressing device configured to surround a limb or joint of a user, according to one of claims 1 to 11, the method comprising steps of assembling elastic band segments (10, 20) to obtain band segments of different stiffness values.
